# EUROPEAN PATENT APPLICATION

(11) **EP 1 362 594 A1**
(43) Date of publication of application: **19.11.2003**
(21) Application number: 02715862.5
(22) Date of filing: 22.01.2002
(51) Int. Cl.: A61K 31/765, A61P 43/00, A61P 1/04, A61P 35/00, A23L 1/30

(54) **ANTI-STRESS AGENTS**

(30) Priority: 24.01.2001 JP 2001016145
(71) Applicant: AMATO PHARMACEUTICAL PRODUCTS, LTD., Fukuchiyama-shi, Kyoto 620-0932 (JP)
(72) Inventor: MURAKAMI, Masahiro, Osaka-shi, Osaka 547-0026 (JP); TAKADA, Shigeo, Isehara-shi, Kanagawa 259-1112 (JP); NAGATO, Yasukazu, Atsugi-shi, Kanagawa 243-0122 (JP); OKABE, Susumu, Kyoto-shi, Kyoto 602-0897 (JP)
(74) Representative: Sternagel, Fleischer, Godemeyer & Partner Patentanwälte
(86) International application number: JP0200411
(87) International publication number: WO02060457

(57) **Abstract**

The object of the present invention is to provide a novel antistress agent which can be used for suppressing stress. According to the present invention, there is provided an antistress agent which comprises a mixture of cyclic and/or straight chain poly lactic acids having a condensation degree of 3 to 20.

## Description

### TECHNICAL FIELD

The present invention relates to an antistress agent. More specifically, the present invention relates to an antistress agent which can be used as a medicament, food for special healty uses, health food and the like, which are effective for preventing and alleviating mental and physical symptoms derived from stress.

### BACKGROUND ART

The modem world is in the environment where scientific technologies are progressing and thickening and a social state is violently changing. Peoples are exposed to various types of physical, chemical, psychological and social stresses. Especially, in internationalized societies, a complicated human relationship has been formed and thus, various symptoms caused by mental stresses have been reported.

It is known that the mental stress influences largely to a circulation system, immune system and the like. There is unknown point in a scientific concept and definition of a stress, and many problems remain in the evaluation of a stress. In recent years, medical examination is being carried out.

For example, it has been reported that upon receiving stress, angiotensin II and the like is increased and sodium content in a living body becomes excessive by reabsorption of sodium to cause an increase of blood pressure (Osamu Mohara; Metabolism 28(2); 323, 1911). In addition, on the basis of these findings, a study has been conducted on the effect of angiotensin conversion enzyme inhibitors, enalapril and alacepril, which are used as a hypertension therapeutic agent, on the hypertension caused by the stress (The American Journal of Cardiology; 68(15) 1362 (1991), International Medicine; 32 (9) 691 (1993)).

In addition, it has been reported that when a brain detects a psychological stress, discharge of noradrenaline progresses in a wide range of brain site to cause such mental symptoms as anxiety and tension (Masatoshi Tanaka, Metabolism Vol. 26: 122-131, 1989). As mechanisms thereof, it has been reported that a corticotropin-releasing factor (CRF) is discharged from a hypothalamus and, in response therewith, secretion of an adrenocorticotropic hormone (ACTH) from a pituitary gland increases, and that corticoid is secreted from an adrenal cortex by ACTH so as to work biophylactic mechanisms (Brabdenberger, G et al. (1980) Biol. Physiol. Vol. 10: 239-252).

On the other hand, the hypothalamus is a center of an autonomous nervous system and hence, stimulates a sympathetic nerve to secrete noradrenaline. It is presumed that the sympathetic nerve exists in an adrenal medulla and stimulation makes secretion of adrenaline in blood, and by action of these catecholamines, physical changes such as an increase of heartbeat and blood pressure and facial flush appears (Dimsdale, JE., Moss, J. (1980) J. Am. Med. Assoc. Vol. 243: 340-342).

When the stress is strong or continues for a long term, the stress gives an influence to various organs of a whole body and as a result, serious psychophysiologic disorder such as digestive ulcer, ischemic heart disease, cerebrovascular disease, hypertension, and hyperlipemia may occur.

As an agent for relieving and preventing the mental and physical symptoms caused by the stress, chemically synthesized drugs such as a tranquilizer, an antianxiety agent and somnifacient are used. However, these drugs have problems of habituation and adverse effects and thus, everyday use thereof with a purpose of prevention of mental and physical symptoms caused by the stress is not preferable. As a symptomatic therapy of mild mental fatigue, relaxation and aromatherapy have been known. However, there is a large individual difference in the effect thereof and thus, a long term and continuous practice is required.

Therefore, it is desired to develop a novel antistress agent which can be repeatedly used, has no problem as to safety, and shows the effect of relief and prevention of mental and physical symptoms caused by the stress.

From studies that have been made so far, it has been reported that a mixture of cyclic and/or straight chain poly L-lactic acids having a condensation degree of 3 to 20 is useful as an antineoplastic agent (Japanese Patent Application Laying-Open (Kokai) Nos. 9-227388 and 10-130153). However, evaluation of stress- suppressing effect of a mixture of cyclic and/or straight chain poly L-lactic acids having a condensation degree of 3 to 20 has not been reported.

### DISCLOSURE OF THE INVENTION

An object of the present invention is to provide a novel antistress agent which can be used for suppressing a stress. More particularly, an object of the present invention is to provide an antistress agent which can be repeatedly used, has high safety, and can relieve and prevent mental and physical symptoms caused by the stress. Further, another object of the present invention is to provide food and drink for suppressing stress.

In order to study for the purpose of solving the aforementioned objects, the present inventors have administered a mixture of cyclic and/or straight chain poly lactic acids having a condensation degree of 3 to 20 to rats and have studied the degree of occurrence of a gastric mucosa injury induced by a water immersion restraint stress. As a result, it has been found that the occurrence of the gastric mucosa injury can be significantly suppressed by administration of the mixture of poly lactic acid.

The present inventors have studied also the effect of the mixture of poly lactic acid on the survival ratio of a model mouse of diabetes which was exposed to cold stress. As a result, it has been found that the survival ratio of a model mouse of diabetes is higher in the case where the mixture of poly lactic acids was administered.

Further, the present inventors have measured the number of lymphocytes, the formation of tumors and the anaerobic enzyme activity in the mouse under stress. As a result, it has been demonstrated that, by administration of a mixture of poly lactic acids, the reduction of the number of lymphocytes and the increase of the anaerobic enzyme activity due to stress are suppressed, and anti-tumor effect is achieved.

The present invention has been completed on the basis of these findings.

Thus, according to the present invention, there is provided an antistress agent which comprises a mixture of cyclic and/or straight chain poly lactic acids having a condensation degree of 3 to 20.

The antistress agent according to the present invention can be used, for example, for preventing and improving a medical symptom caused by the stress, and more specifically can be used for preventing and improving deterioration of an immune function which is caused by the stress, for preventing and improving an ulcer or a tumor caused by the stress, and for improving the survival ratio against the stress of a patient having a disease.

Preferably, the lactic acid that is a repeating unit in the poly lactic acid consists substantially of L-lactic acid.

Preferably, the mixture of cyclic and/or straight chain poly lactic acids having a condensation degree of 3 to 20 is a fraction obtained by condensing lactic acids by dehydration under an inactive atmosphere, subjecting the ethanol- and methanol-soluble fractions of the obtained reaction solution to reverse phase column chromatography, and eluting with 25 to 50 weight % acetonitrile aqueous solution of pH 2 to 3 and then with 90 weight % or more acetonitrile aqueous solution of pH 2 to 3.

Preferably, condensation by dehydration is performed by stepwise decompression and temperature rise under nitrogen gas atmosphere.

Preferably, reverse phase column chromatography is performed by ODS column chromatography.

According to another aspect of the present invention, there are provided food and drink for suppressing stress which comprises the aforementioned antistress agent of the present invention.

According to still another aspect of the present invention, there is provided the use of a mixture of cyclic and/or straight chain poly lactic acids having a condensation degree of 3 to 20 in the production of an antistress agent or food and drink for suppressing stress.

According to a still further aspect of the present invention, there is provided a method for suppressing stress, which comprises a step of administering an effective amount of a mixture of cyclic and/or straight chain poly lactic acids having a condensation degree of 3 to 20 to mammals such as humans.

### BRIEF DESCRIPTION OF THE DRAWINGS

Figure 1 shows a mass spectrum of the mixture of poly lactic acids obtained by Production Example 1.
Figure 2 is a graph showing the influence of CPL on the development of a gastric mucosa injury induced by a water immersion restraint stress in rat.
Figure 3 is a graph showing the effect of CPL on the survival ratio of a diabetes model mouse exposed to a cold stress.
Figure 4 is a graph showing the result of measurement of a fasting blood glucose level of a diabetes model mouse.

### THE BEST MODE FOR CARRYING OUT THE INVENTION

The embodiment and method for the practice of the present invention are described in detail below.

The antistress gent of the present invention comprises, as an active ingredient, a mixture of cyclic and/or straight chain poly lactic acids having a condensation degree of 3 to 20, and can be used for relieving and preventing mental and physical symptoms caused by the stress.

The cause of the stress (stressor) which is a target of use of the antistress agent according to the invention is not specially limited. The stressor includes, for example, physical environmental factors (temperature, noise, earthquake, etc.), social and cultural factors (war, economic crisis, coup, etc.), changes (movement, school transfer, turning employment, retirement, etc.) of personal environmental conditions (home, school, office, etc.), and biological factors such as discord and conflict in human relationship as well as overfatigue, injury, physical disease, and the like. It is understood that in many psychophysiologic disorder observed in today, these stressors is a direct cause of disease onset or takes an important role on its onset.

A stress is thought to be a mental and physical distortion, which occurs when a causal factor of the stress as described above (e. g., physical signs such as diseases such as pain and onset of fever, and disorders; mental signs such as tension, anxiety and fear caused by human relationship in a society and a home; and physical environmental factors such as a noise, a meteorological change) is applied to a living body.

The living body shows a reaction specific to the stimulus which causes any disturbance. At the same time, regardless of the type of the stimulus, the activities of the sympathetic nerve-adrenal medulla system and a pituitary gland-adrenal cortex system are increased, and adrenaline is secreted so as to raise the blood pressure and increase the pulse rate and the respiration rate. On the other hand, the secretion of a hormone named cortisol from adrenal cortex is increased and the living body shows the reaction of protection or adaptation. This reaction is named a stress reaction, and it is known that the reaction occurs through the following mechanisms. When the living body is subjected to a physical or mental stressor, its information reaches the central nervous system and extends to the hypothalamus. A direction of a stress reaction is given therefrom. Transmission thereof is performed through major two routes. One of them is through the sympathetic nerve system and a larger part of the sympathetic nerve system itself reaches various organs in the living body to discharge noradrenaline from a peripheral of nerve to express the effect. A smaller part thereof reaches the adrenal medulla to stimulate it to make secrete adrenaline. Through the other stress information transmission route, a hormone (CRH) which stimulates secretion of ACTH hormone by the pituitary gland is secreted from the hypothalamus, resulting in the secretion of ACTH from the pituitary gland. ACTH is transferred to the adrenal cortex by blood and then, stimulates the adrenal cortex to promote secretion of the hormone named cortisol.

The stress reaction plays a useful role of a protection reaction for the living body. It is presumed that too much cortisol and adrenaline are secreted in the reaction and hence act harmfully to many organs resulting in impossible maintenance of homoeostasis of the living body to cause a stress disease. That is, excess of cortisol breaks the gastric and duodenal mucosae to cause a gastroduodenal ulcer and suppress the immune function. Therefore, a resistance to infection becomes lower and cancer becomes easy to occur. The excess of adrenaline makes hypertension and circulatory diseases easy to occur.

In the case where the antistress agent according to the invention is administered to a subject, the change of a physiological index such as deterioration of the immune function, which occurs in response to a stress application, becomes small in comparison with the case of no administration. Therefore, it is understood that the antistress agent according to the invention has an effect of reducing the stress of the subject patient, and can prevent onset of the medical symptom caused by various stresses to which the patient is subjected in everyday life, and can relieve and alleviate the symptom.

As medical symptoms caused by the stress include lowering of the immune function, occurrence of ulcer or tumor, lowering of the survival ratio of disease patients.

The specific medical symptoms caused by the stress are exemplified by circulatory diseases such as arterial sclerosis, ischemic heart disease (cardiac angina, cardiac infarction), essential hypertension, cardiac neurosis and allorhythmic pulse; respiratory system diseases such as bronchial asthma, hyperventilation syndrome and nervous coughing; digestive system diseases such as peptic ulcer, ulcerative colitis, irritable bowel syndrome, anorexia nervosa, nervous vomiting, abdominal distension and aerophagia; endocrine metabolic system diseases such as adiposis, diabetes, compulsive water drinking and Basedow's disease; nervous system diseases such as migraine, muscle contraction headache and autonomic dystonia; urinary system diseases such as nocturnal enuresis, impotence and neurogenic bladder; bone muscle system diseases such as chronic arthrorheumatism, systemic myalgia and rachiodynia; dermal system diseases such as nervous dermatitis, alopecia areata, hidrosis and eczema; otolaryngological field diseases such as labyrinthine syndrome, laryngopharyngeal foreign body sensation, auditory disorder, buzzing in the ear, car sickness, and aphonia and stutter; ophthalmologic field diseases such as primary glaucoma, eye fatigue, blepharospasm and eye hysteria; obstetrics and gynecologic field diseases such as dysmenorrhea, amenorrhea, emmeniopathy, dysfunctional uterine bleeding, climacteric disorder, dyspareunia and infertility; pediatrics field diseases such as orthostatic circulation disorder, recurrent umbilical colic, nervous fever and night terror; pre- and post-operative conditions such as intestinal adhesion, dumping syndrome, polysurgery and neurosis after plastic surgery; stomatologic field diseases such as cataplectic glossalgia, a certain stomatitis, oral dysodia, abnormal salivation, masseter muscle check bite and artificial tooth neurosis; neurosis; depression; and oncogenesis.

The antistress agent according to the invention has a preventive action that an uptake of the agent before subjecting to the stress makes a person difficult to subject to the stress and even if the person is subjected to the stress, makes prevention of onset of a medical symptom easy. Hence, it is preferable to take daily the antistress agent according to the invention as a health food or a drug.

In the antistress agent and the food and drink for suppressing stress according to the present invention, a mixture of cyclic and/or straight chain poly lactic acids having a condensation degree of 3 to 20 is used as an active ingredient.

The term "a mixture of poly lactic acids" used in the present invention means a mixture wherein cyclic and/or straight chain poly lactic acids having a condensation degree of 3 to 20 are present at any ratio. That is to say, the term "mixture" does not only mean a mixture of poly lactic acids having any condensation degree ranging from 3 to 20, but is also used as a concept including a mixture of cyclic and straight chain poly lactic acids. As is described below in the present specification, "a mixture of poly lactic acids" can be obtained by condensing lactic acids by dehydration and then performing purification by a suitable method. Although the term "a mixture of poly lactic acids" is used in the present specification for the sake of convenience, this term also includes a poly lactic acid consisting of a single ingredient such as a cyclic poly lactic acid having single condensation degree or a straight chain poly lactic acid having single condensation degree.

The term "condensation degree" is used to mean the number of lactic acid unit that is a repeating unit in poly lactic acids. For example, the cyclic poly lactic acid is assumed to have the following structural formula wherein n represents condensation degree (n = 3 to 20).

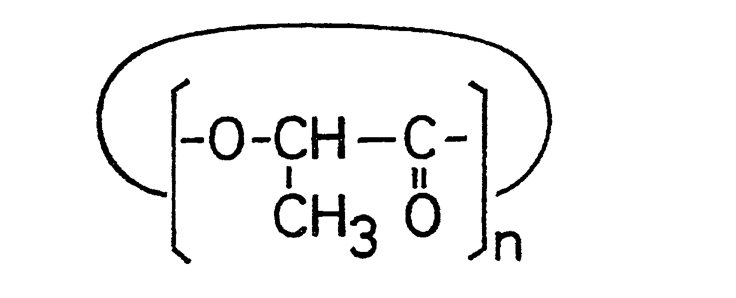

When "lactic acid" is simply referred to in the present specification, this lactic acid includes all of L-lactic acid, D-lactic acid or a mixture comprising these types of lactic acid at any ratio. Preferably in the present invention, the lactic acid consists substantially of L-lactic acid. The term "substantially" is used herein to mean that the ratio of L-lactic acid units in a mixture of poly lactic acids (number of L-lactic acid unit / number of L-lactic acid unit + number of D-lactic acid unit x 100) is, for example, 70% or more, preferably 80% or more, more preferably 85% or more, further more preferably 90% or more, and particularly preferably 95% or more. The ratio of L-lactic acid units in a mixture of poly lactic acids depends on the ratio of L-lactic acid and D-lactic acid that exist in lactic acids used as a starting substance.

The methods for producing a mixture of cyclic and/or straight chain poly lactic acids having a condensation degree of 3 to 20 are not particularly limited, and the mixture of poly lactic acids can be obtained by the production methods described, for example, in Japanese Patent Application Laying-Open (Kokai) Nos. 9-227388 and 10-130153 or Japanese Patent Application No. 11-39894 (All publications cited herein are incorporated herein by reference in their entirety).

More specifically, for example, a mixture of cyclic and/or straight chain poly lactic acids having a condensation degree of 3 to 20 can be obtained by the following method A.

### Method A:

First, lactic acid (preferably, lactic acid substantially consisting of L-lactic acid) is condensed by dehydration under an inactive atmosphere. Examples of the inactive atmosphere include nitrogen gas and argon gas, and nitrogen gas is preferred.

Dehydration and condensation reaction is carried out at a temperature of 110°C to 210°C, preferably 130°C to 190°C under normal pressure to reduced pressure of approximately 1mmHg, and particularly preferably the reaction is carried out by stepwise decompression and stepwise temperature rise. A reaction period can be determined as appropriate. For example, the reaction can be carried out for 1 to 20 hours. Where stepwise decompression and stepwise temperature rise are applied, reaction is performed by dividing the reaction period into two or more partial reaction periods, and then determining pressure and temperature for each of the reaction periods. Where stepwise decompression is applied, pressure can be reduced, for example, from a normal pressure to 150mmHg and then to 3mmHg. Where stepwise temperature rise is applied, temperature can be raised, for example, from 145°C to 155°C and then to 185°C. Practically, the reaction can be carried out by using these conditions in combination, for example, 145°C, normal pressure, 3 hours; 145°C, 150mmHg, 3 hours; 155°C, 3mmHg, 3 hours; and 185°C, 3mmHg, 1.5 hours.

Subsequently, ethanol and methanol are added to the reaction mixture obtained by the dehydration and condensation reaction, and the mixture is filtered. The obtained filtrate is dried to obtain ethanol- and methanol-soluble fractions. The term "ethanol- and methanol-soluble fractions" is used in the present specification to mean fractions soluble in a mixed solution of ethanol and methanol. In order to obtain ethanol and methanol-soluble fractions, a reaction mixture obtained by dehydration and condensation reaction is mixed with ethanol and methanol, where the ratio of ethanol and methanol can be determined as appropriate. For example, the ratio is ethanol:methanol = 1 : 9. The order, method and the like for adding ethanol and methanol to a reaction mixture are not limited, and may be selected as appropriate. For example, ethanol may be added at first to the reaction mixture obtained by the dehydration and condensation reaction, and then methanol may be added thereto.

The thus obtained ethanol- and methanol-soluble fractions are subjected to reverse phase column chromatography, especially to chromatography where an octadecylsilane (ODS) column is used. First, fractions eluted with 25 to 50 weight % acetonitrile aqueous solution of pH 2 to 3 are removed, and then fractions eluted with 90 weight % or more acetonitrile aqueous solution of pH 2 to 3, preferably 99 weight % or more acetonitrile aqueous solution, are collected so as to obtain a mixture of cyclic and/or straight chain poly lactic acids having a condensation degree of 3 to 20.

The thus obtained mixture of cyclic and/or straight chain poly lactic acids is neutralized with an alkaline substance such as sodium hydroxide, and is dried under reduced pressure, and then according to standard techniques, the mixture can be formulated in a desired form as mentioned below.

Other examples of the methods for producing a mixture of cyclic and/or straight chain poly lactic acids having a condensation degree of 3 to 20 used in the present invention include a method described in Japanese Patent Application No. 11-265715 (hereinafter referred to as method B), or a method described in Japanese Patent Application No. 11-265732 (hereinafter referred to as method C) (All publications cited herein are incorporated herein by reference in their entirety). Methods B and C will be described specifically below.

### Method B:

Method B is a method for producing a cyclic lactic acid oligomer which comprises polymerizing lactid in the presence of a lithium compound represented by RYLi [wherein R represents an aliphatic group or aromatic group, Y represents oxygen atom or sulfur atom]. In the case of performing the polymerization reaction, the ratio of the amounts of the lithium compound (RYLi) is 1-0.1 mol, preferably 0.2-0.3 mol per mol of lactide. The reaction temperature is -100 to 0°C, preferably -78 to -50°C. Reaction is preferably carried out by starting from a temperature of -78 to -50°C and gradually raising it to room temperature. The reaction is preferably carried out in the presence of a reaction solvent. As the reaction solvent, there can be used, for example, a cyclic ether such as tetrahydrofuran, diethylether, and dimethoxyethane. The reaction atmosphere can be an inactive gas atmosphere such as nitrogen gas and argon. The reaction pressure is not limited, and is preferably a normal pressure.

The composition (that is, the mixing ratio of cyclic lactic acid oligomer and a chain lactic acid oligomer) of the lactic acid oligomer obtained as described above fluctuates depending on the lithium compound used as a reaction assistant. Where a lithium compound of alkyl alcohol having a carbon number of 1 to 3 (ROLi) (wherein R represents an alkyl group with carbon number 1 to 3) is used as a lithium compound, a mixture of a cyclic lactic acid oligomer and a chain oligomer (proportion of the cyclic lactic acid oligomer: 80 to 85 weight %) is obtained. When a lithium compound of alkyl alcohol having a carbon number of 4 or more such as t-butyl alcohol, or thiophenol compound is used as a lithium compound, substantially only a cyclic lactic acid oligomer can be selectively obtained.

### Method C:

This method comprises:
(i) a first heating step which comprises heating lactic acid under a pressure condition of 350 to 400 mmHg and to a temperature of 120 to 140°C so as to perform dehydration and condensation, and distilling off and removing only by-product water without distilling lactid off;
(ii) a second heating step for synthesizing a product condensed by dehydration comprising chain lactic acid oligomers as the main ingredient, which comprises, after completion of the first heating step, heating the reaction product to a temperature of 150 to 160°C while reducing the reaction pressure to 15 to 20 mmHg at a decompression rate of 0.5 to 1 mmHg/min, wherein only by-product water is distilled off and removed while avoiding distillation of lactid; and after the reaction pressure is reduced to 15 to 20 mmHg, maintaining the reaction under the same pressure condition and at a reaction temperature of 150 to 160°C;
(iii) a third heating step for synthesizing cyclic oligomers which comprises, after completion of the second heating step, heating under a pressure condition of 0.1 to 3 mmHg and at 150 to 160°C to cyclize the chain lactic oligomer.

In this method, first, in the first heating step, lactic acid is heated under reduced pressure to perform dehydration and compression reaction. In this case the reaction period is 3 to 12 hours, preferably 5 to 6 hours. To allow the reaction in the first heating step to proceed smoothly, by-product water produced by condensation of lactic acids by dehydration is distilled off. At this time, distillation of by-product water is performed such that lactid, which is the dehydrated condensed product of two molecules of lactic acid, is not distilled off. To achieve such purpose, the reaction pressure is maintained at a reduced pressure, preferably 300 to 500 mmHg, more preferably 350 to 400 mmHg. Under this pressure condition, heating is performed at a temperature range of 100 to 140°C, preferably 130 to 140°C. The reaction product produced by reaction in the first heating step mainly comprises as the main ingredient a dehydrated condensed product of 3 to 23 molecules of lactic acid.

To obtain oligomers having an increased average degree of polymerization in the second heating step after completion of the above first heating step, heating is performed at a temperature higher than the reaction temperature of the above first heating step, preferably at 145°C to 180°C, more preferably 150°C to 160°C, while the reaction pressure is reduced to 10 to 50 mmHg, preferably 15 to 20 mmHg, so that dehydration and condensation reaction is further continued.

As with the reaction in the above first heating step, reaction is performed under a condition where by-product water, but not lactid, is distilled off, to allow the reaction to proceed smoothly. The rate at which reaction pressure is reduced to a pressure in the above range (decompression rate) is normally required to be maintained within a range of 0.25 to 5 mmHg/min, preferably 0.5 to 1 mmHg/min, in order to avoid distillation of lactid and increase the reaction efficiency. A decompression rate lower than the above range is not preferred because it will increase the time required to reduce pressure to a given pressure. On the other hand, a decompression rate higher than the above range is also not preferred because it will cause lactid to be distilled off together with by-product water.

After the reaction pressure is reduced to a certain pressure, reaction is further continued at that reaction pressure. The heating time period in this case is 3 to 12 hours, preferably 5 to 6 hours.

A lactic acid oligomer having an average polymerization degree of 3 to 30, preferably 3 to 23 is obtained by the reaction in the above second heating step. The proportion of cyclic oligomers in the oligomers in this case is normally about 70 to 80 weight %.

In the third heating step, after completion of the above second heating step, a reaction pressure is maintained at 0.25 to 5 mmHg, preferably 0.5 to 1 mmHg, and reaction is further continued at a temperature of 145 to 180°C, preferably 150 to 160°C. A reaction period is 3 to 12 hours, preferably 5 to 6 hours. By-product water produced in this case is also distilled off. In this case, distillation of lactid is preferably avoided. However, since the reaction product contains almost no lactid, it is not required to specially lower the decompression rate.

Lactic acid oligomers produced by reaction in the above third heating step have an average polymerization degree of 3 to 30, preferably 3 to 23, and contain cyclic oligomer in the proportion of 90 weight % or more, preferably 99 weight % or more.

The above methods A, B and C merely show some of specific examples of methods of producing a mixture of poly lactic acids used in the present invention. A mixture of poly lactic acids which is produced by other methods can also be used in the present invention.

The effect of the antistress agent according to the present invention can be confirmed, for example, by using an experimental animal and causing the gastric mucosa injury by means of the water immersion restraint stress induction method. Specifically, an experimental animal (e.g., rat) is placed in a stress cage, immersed in a vessel up to a height of a xiphistemum at a temperature ranging from 22°C to 23°C for 10 hours to give the stress. A drug is given before the stress is applied. Then, the experimental animal is subjected to laparotomy under etherization, a stomach is resected, and a cardia of the stomach is ligated followed by injection of 2% formalin solution from a duodenum into the stomach to fix it. A tunica serosa ventriculi is also immersed in the solution to be fixed. Subsequently, a greater curvature of the stomach is dissected to measure a length of the gastric mucosa injury. A size of the gastric mucosa injury smaller than that of the control group proves the effect of the antistress agent according to the present invention.

The effect of the antistress agent according to the present invention can also be confirmed by measuring a variation of the lymphocyte count in the experimental animal which was subjected to a confined space-restraint stress. Specifically, the experimental animal (e.g., mouse) is kept by housing in an acrylic case and received a solid diet containing the antistress agent. Once a week after start of giving the solid diet, the mouse is placed in a thin cylinder for a certain period to subject it to the confined space-restraint stress for 4 weeks (4 times). Immediately after completion of restraint, blood is collected from a caudal vein under diethyl etherized anesthesia, and the number of lymphocytes is counted under an optical microscope. When the reduction of the lymphocyte count by the stress is suppressed, the effect of the antistress agent according to the present invention can be confirmed.

The antistress agent of the present invention can be prepared by optionally selecting and using a component or an additive used in the formulation of medicaments, quasi-drugs and the like, if necessary, without impairing the effect of the present invention in addition to the aforementioned essential component. The antistress agent of the present invention can be used as single medicaments, and also can be contained and used in medicaments, quasi-drugs and the like.

The dosage form of the antistress agent of the present invention is not particularly limited, and any form suitable for the purpose can be selected from dosage forms for oral or parenteral administration.

Examples of dosage forms suitable for oral administration include a tablet, a capsule, a powder, a drink, a granule, a parvule, a syrup, a solution, an emulsion, a suspension, a chewable tablet, and the like. Examples of dosage forms suitable for parenteral administration include, but are not limited to, an injection (e.g. a subcutaneous, intramuscular or intravenous injection, and the like), an external preparation, a drop, an inhalant, an air spray, dose drops, eye drops.

Liquid formulations suitable for oral administration such as a solution, emulsion or syrup can be produced using water; sugars such as sucrose, sorbit or fructose; glycols such as polyethylene glycol or propylene glycol; oils such as sesame oil, olive oil or soybean oil; antiseptics such as p-hydroxybenzoate; and flavors such as strawberry flavor and peppermint. In order to produce solid formulations such as capsule, tablet, powder or granule, there can be used an excipient such as lactose, glucose, sucrose or mannite; a disintegrator such as starch or sodium alginate; a lubricant such as magnesium stearate or talc; a binder such as polyvinyl alcohol, hydroxypropylcellulose or gelatin; a surfactant such as fatty acid ester; and a plasticizer such as glycerine, and the like.

Formulations for an injection or drop that is suitable for parenteral administration preferably comprise, as an active ingredient, the above substance in a dissolved or suspended state in a sterilized aqueous medium which is isotonic to the recipient's blood. For example, in the case of an injection, a solution can be prepared using an aqueous medium consisting of a saline solution, a glucose solution or a mixture of a saline solution and a glucose solution. In the case of a formulation for intestinal administration, it can be prepared using carriers such as theobroma oil, hydrogenated lipids or hydrogenated carboxylic acid, and can be provided as a suppository. In order to produce an air spray, the above substance as an active ingredient may be dispersed as microparticles, and a carrier which does not irritate the recipient's cavitas oris and respiratory tract mucosa and which facilitates absorption of the active ingredient can be used. Specific examples of carriers include lactose, glycerine, and the like. Formulations having a form such as aerosol or dry powder may be prepared depending on the properties of the substance of an active ingredient and the carrier to be used. One or two or more auxiliary ingredients selected from glycols, oils, flavors, an antiseptic, an excipient, a disintegrator, a lubricant, a binder, a surfactant, a plasticizer and the like may be added to these formulations for parenteral administration.

The dose and dosage frequency of the antistress agent of the present invention are determined as appropriate, depending on various factors such as purpose of administration, dosage form, condition such as age, body weight or sex of a patient. Generally, the dose of an active ingredient per day is 1 to 10,000 mg/kg, preferably 10 to 2000 m/kg, and more preferably 10 to 200 mg/kg. It is preferred that the above dose of the agent is dividedly applied about once to 4 times per day.

The time of administration of the antistress agent of the present invention is not particularly limited. By administering the agent when stress loading is predicted or at the time of stress loading, the stress can be prevented or alleviated. Also, by regularly using the agent, the stress can be prevented or alleviated.

The present invention also relates to food and drink for suppressing stress which comprises a mixture of cyclic and/or straight chain poly lactic acids having a condensation degree of 3 to 20. Thus, the mixture of cyclic and/or straight chain poly lactic acids having a condensation degree of 3 to 20 which is used in the present invention is used not only as a form of single agent as mentioned above, but also may be mixed in food and drink, and used.

The preparation form of the food and drink for suppressing stress according to the present invention is not particularly limited, so long as a mixture of poly lactic acid can be contained without being decomposed.

Specific examples of products of the food and drink for suppressing stress according to the present invention include health foods or supplements including drinks, such as those generally called a soft drink, drinkable preparation, health food, specified supplement food, functional food, function-activating food, nutritional supplementary food, supplement, feed, feed additive and the like.

Specific examples of food and drink include confectionary, such as a chewing gum, chocolate, candy, sweet tablet, jelly, cookie, biscuit and yogurt; frozen deserts, such as ice cream and sherbet; beverages, such as tea, soft drink (including juice, coffee, cocoa and the like), nutrition supplement drinkable preparation, and cosmetic drinkable preparation; and all other food and drink, such as bread, ham, soup, jam, spaghetti, and frozen food. Alternatively, the mixture of poly lactic acids used in the present invention can also be used by adding to seasoning, food additives, and the like. By the use of the food and drink of the present invention, there can be provided safe food and drink which can exert stress-suppressing effect and show substantially no toxic side effect.

The food and drink for suppressing stress according to the present invention can be obtained by directly mixing and dispersing a mixture of poly lactic acids in a common raw material used in food, and then processing the mixture into a desired form by a know method.

The food and drink for suppressing stress according to the present invention encompasses food and drink in every form, and the types are not specifically limited. That is, the food and drink can be provided by mixing the antistress agent of the present invention into the above-mentioned various food and drink, or various nutrient compositions, such as various oral or enteral nutrient preparations or drinks. Compositions of such food and drink may include protein, lipid, carbohydrate, vitamin and/or mineral, in addition to the mixture of cyclic and/or straight chain poly lactic acids having a condensation degree of 3 to 20. The form of the food and drink is not specifically limited, and may be in any form, such as solid, powdery, liquid, gel, and slurry forms, so far as it is in a form that is easily ingested.

The content of the mixture of poly lactic acids in the food and drink is not specifically limited, and is generally 0.1 to 20 weight %, more preferably approximately 0.1 to 10 weight %.

The mixture of poly lactic acids is preferably contained in the food and drink in an amount which achieve a stress-suppressing effect which is an object of the present invention. Preferably, about 0.1 g to 10 g, more preferably about 0.5 g to 3 g, of the mixture of poly lactic acids is contained per food or drink to be ingested.

The content of the specification of Japanese Patent Application No.2001-16145 which the present application claims a priority based on is incorporated herein by reference as a part of the disclosure of the present specification.

The present invention is further described in the following examples, but the scope of the present invention is not limited by the examples in any way.

### EXAMPLES

### Production Example 1: Production of a mixture of poly lactic acids (hereinafter referred to as CPL)

500 ml of L-lactic acid (to which D-lactic acid was also mixed) was placed into a separable flask in a mantle heater. 300ml/min of nitrogen gas was flowed therein while stirring. Accumulated water was introduced into a flask equipped with a reflux condenser via a warmed descending type connecting tube, while heating at 145°C for 3 hours. Furthermore, after pressure was reduced to 150 mmHg and heated at the same temperature for 3 hours, the mixture was heated at 155°C for 3 hours under a reduced pressure of 3 mmHg, and then at 185°C for 1.5 hours under a reduced pressure of 3 mmHg to obtain poly lactic acids as a reaction product.

The obtained poly lactic acids were kept at 100°C, and 100ml of ethanol and 400ml of methanol were separately added thereto, and then the mixture was allowed to be cooled. This mixture was added to 500ml of methanol, and the mixture was well stirred and left to stand. Then, the mixture was filtrated for purification. The filtrate was subjected to vacuum drying and then dissolved in acetonitrile to obtain 200ml (stock solution) in total.

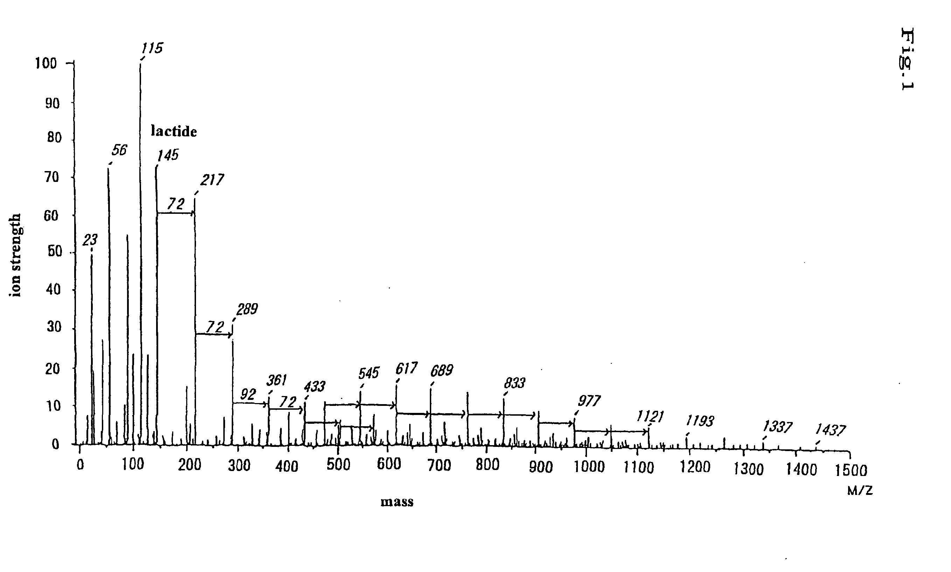

The stock solution was subjected to a reverse phase ODS column (TSK gel ODS-80 TM) which was previously equilibrated, and was stepwise eluted with 30%, 50% and 100% acetonitrile (pH2.0) each containing 0.01M hydrochloric acid to obtain poly lactic acids (condensation degree of 3 to 20) as an acetonitrile 100% elution fraction. The mass spectrum of the obtained substance is shown in Fig. 1. As is clear from the regular fragment ion peaks in Fig. 1, the obtained mixture of poly lactic acids mainly comprises cyclic condensate, and a small amount of linear condensate is contained therein.

### Test Example 1: Effect of CPL on the gastric mucosa injury induced by the water immersion restraint stress in model rat

### (Materials and method)

For the experiment were used 6-week-old male Donryu line rats (SLC Japan, a body weight at the experiment was 180 to 200 g).

CPL was dissolved in glycerin which was warmed to 40°C and then, propylene glycol was added to adjust the volume to 100 mg/ml. Thereafter, the solution was diluted with distilled water to make 50 mg/kg dose. The drug was orally administered once a day for 10 days in a proportion of 1 ml/200g of body weight. In the control group, only solvent was given orally in the same dose.

The gastric mucosa injury induced by the water immersion restraint stress was prepared as follows. The rat was fasted for 18 hours before the start of the experiment, allowed to take water freely 2 hours before the start of the experiment followed by subsequent stopping of water uptake. The rat was placed in a Toudai Yakusa type stress cage (Natsume Seisakusyo CO., LTD) and immersed in the vessel up to the height of the xiphisternum at the temperature ranging from 22°C to 23°C for 10 hours, thereby giving the stress to the rat. The drug was again given at 30 minutes before the stress was applied.

An area of the gastric mucosa injury was measured by the following steps. The rat was subjected to laparotomy under etherization, and the stomach was resected. The cardia of the stomach was ligated followed by injection of 8 ml of the 2% formalin solution from the duodenum into the stomach to fixed it for 10 minutes. The tunica serosa ventriculi is also immersed in the solution to be fixed for 10 minutes. Subsequently, the greater curvature of the stomach was dissected to measure the length (mm) of the gastric mucosa injury using a stereoscopic microscope.

The result was shown in mean±standard error (mean±S. E) (N=10 to 11). A statistical significant of difference was tested by applying Student's t test and P<0.05 was regarded to be significant.

### (Result)

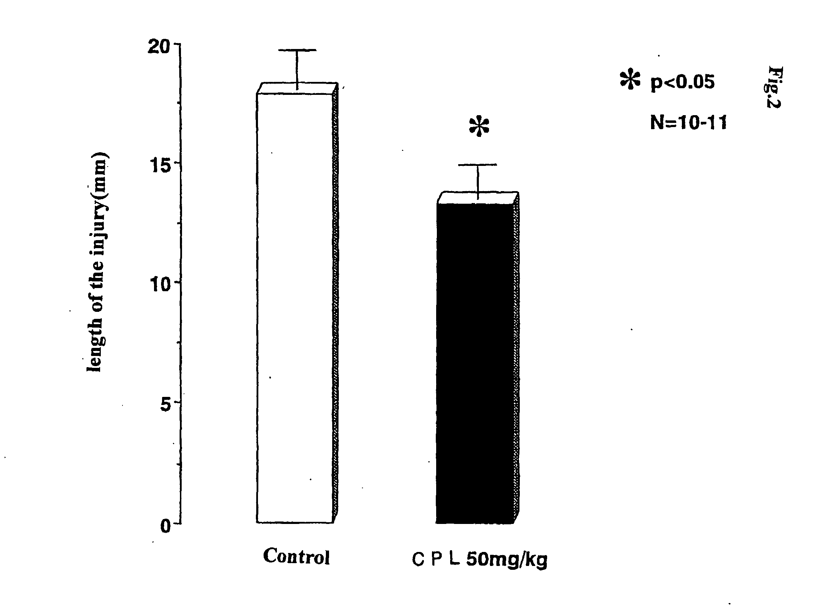

The result of the measurement is shown in the following Table 1 and Fig. 2.

**Table 1.**

| Gastric mucosa injury (mm) | | |
|---|---|---|
| | Control | CPL(50mg/kg) |
| 1 | 20.0 | 8.0 |
| 2 | 22.0 | 19.0 |
| 3 | 20.0 | 6.0 |
| 4 | 19.0 | 16.0 |
| 5 | 15.0 | 18.0 |
| 6 | 16.0 | 15.0 |
| 7 | 12.0 | 10.0 |
| 8 | 12.0 | 16.0 |
| 9 | 15.0 | 10.0 |
| 10 | 28.0 | 15.0 |
| 11 | | 11.0 |
| Mean | 17.9 | 13.1 |
| Standard division | 1.6 | 1.3 |

As shown in Table 1 and Fig. 2, the mean of a sum of lengths of injuries induced by stress loading was 17.9±1.6 mm. On the contrary, the occurrence of the gastric mucosa injury induced by the water immersion restraint stress during repeated administration of CPL for 10 days was significantly suppressed and the mean of the sum of lengths of injuries was 13.1 ± 1.3 mm.

### Test Example 2: Effect of CPL on the survival ratio of diabetes model mouse subjected to cold stress

### (Method)

A special solid diet (1% CPL feed), that has the same nutrient content as that of a standard solid diet CE2 and contains 1% CPL, was prepared by CLEA JAPAN INC.

Six-week-old female mice (C57-db/db) with the type II diabetes were purchased from CLEA JAPAN INC. Immediately after purchasing, the mice were divided into two groups of CE2 group to be fed with the standard solid diet CE2 and the CPL group to be fed with a special diet (1% CPL diet). For water, tap water was freely taken. Heat-dried flooring material, "Clean Chip", made by CLEA JAPAN INC. for the experimental animal was placed on a floor and was replaced with new one everyday.

The diet was not given at 18:00 in the day before measurement of blood glucose and blood was collected between 08:30 to 09:30 in the day of measurement. After blood collection, 0.12 ml of 5% TCA was added to 0.01 ml blood, and the mixture was stirred and centrifuged at 10,000 rpm for 5 minutes. The resultant supernatant was used as a sample. Blood glucose was measured by measuring glucose by o-toluidine-boric acid method.

A systemic calorimeter "BioDynamic Calorimeter BDC-200" made by ESCO, Ltd. (Densi Kagaku K. K.) was employed as a colorimeter to measure discharged heat quantity. The mouse was placed in the systemic colorimeter at 09:00 and taken out at 17:00 in the next day. The diet and water were freely given to the mouse during calorie measurement.

### (Result)

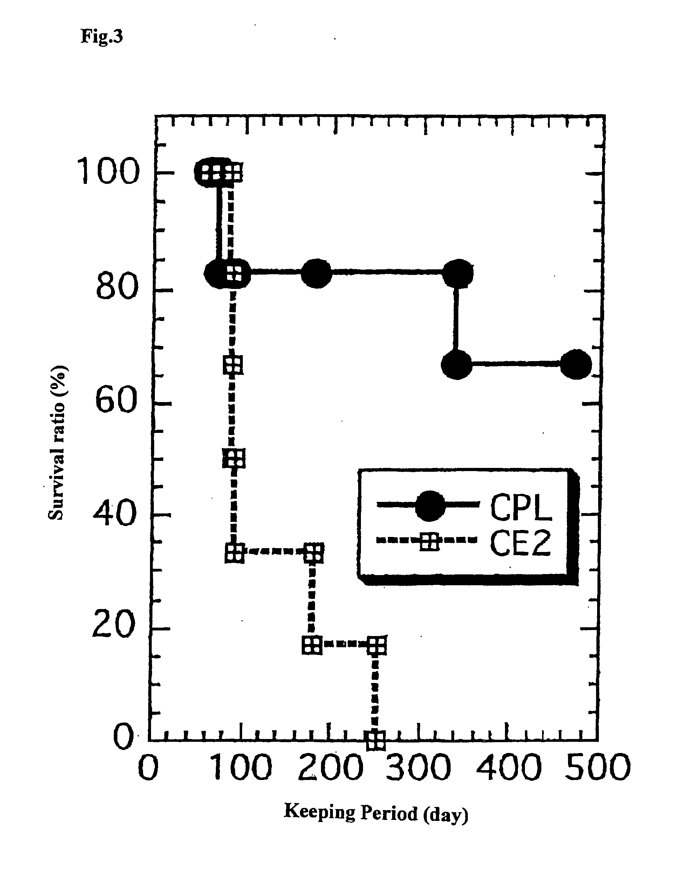

The effect of CPL on the survival ratio of the diabetes model mouse subjected to cold stress is shown in Fig. 3. In the CE2 group, 4 of 6 mice died due to the exposure to cold through calorie measurement for 31 hours, which was conducted in the 91st day after animal keeping was started. The remaining 2 mice died in the 181st and the 254th days after animal keeping was started. In the CPL group, 1 of 6 mice died due to the exposure to cold through calorie measurement for 31 hours, which was conducted in the 82st day after animal keeping was started, and one died in the 242th days after animal keeping was started, but the remaining 4 animals are alive.

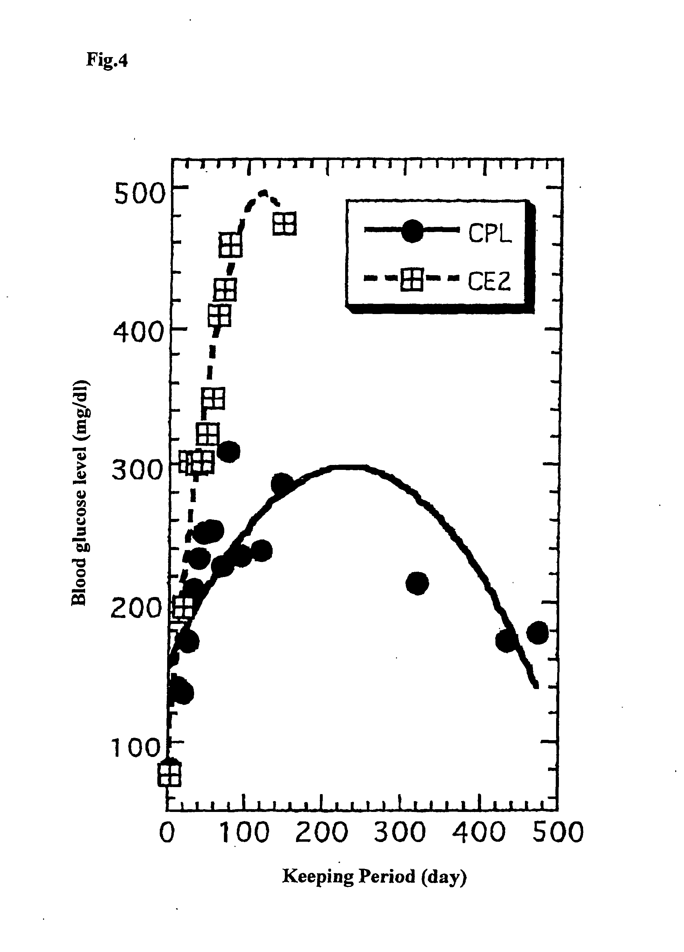

Fig. 4 shows the result of measurement of fasting blood glucose. The mean fasting blood glucose of 4 living animals of the CPL group was 210 mg/dl in the 310th day after animal keeping was started. The mean fasting blood glucose was 170 mg/dl in the 432nd day after animal keeping was started.

Table 2 shows the fasting blood glucose of 4 living animals of the CPL group. The fasting blood glucose of 3 of 4 animals in the 472nd day after animal keeping was started was greatly lower than the fasting blood glucose in the 78th day after animal keeping was started. Particularly, the fasting blood glucose of db/db (4) and (6) showed a normal value. No urinary sugar of these 2 animals was detected after feeding.

**Table 2.**

| Long term effect of CPL on the fasting blood glucose | | | | |
|---|---|---|---|---|
| Animal | Period of keeping (Days) | | | |
| | 78 | 148 | 432 | 472 |
| db/db(1) | 371 | 277 | 209 | 162 |
| db/db(4) | 434 | 340 | 176 | 99 |
| db/db(5) | 362 | 389 | 221 | 354 |
| db/db(6) | 202 | 168 | 84 | 91 |

Table 3 shows the effect of CPL on the discharged heat. A mean discharged heat of the CPL group in the 140th day after animal keeping was started was 32.0 kJ/day which was 77% of the db/- group. The mean discharged heat in the 480th day after animal keeping was started was 41.5 kJ/day, which was equal to the value of the db/group in the 140th day after animal keeping was started.

**Table 3.**

| Effect of CPL on the discharged heat (free heat) | | |
|---|---|---|
| | 140th day | 480th day |
| db/-, CE2(n=4) | 41.3±4.0 | |
| db/db, CE2(n=6) | 22.5±7.7 | |
| db/db, CPL(n=6) | 32.0±4.8 | 41.5±10.2(n=4) |

As described above, the survival period of the db/db mice which was kept using the standard diet containing no CPL was short, and all 6 animals died at about 10th month after birth. In the group which was kept with the diet containing 1% CPL, 4 of 6 animals are alive. The fasting blood glucose of 2 of the 4 mice which are alive is within a range of the normal value, and urinary sugar was not detected. Namely, type II diabetes was cured. The high blood glucose in 1 of 4 mice was improved to be an almost normal value, and urinary sugar was detected in a very small value.

### Test Example 3. Effect of CPL on the change of lymphocyte count in the mouse subjected to the confined space-restraint stress

### (method)

Four-week-old male CBA/ JNCr mice were kept by housing them in the acrylic case.

The mice were divided into the control group which received freely the standard solid diet CE2 (obtained from CLEA JAPAN INC.) and the CPL group which received freely the standard solid diet CE2 containing 0.1% by weight of a mixture of poly lactic acid in the above described standard solid diet (prepared by CLEA JAPAN INC.). After start of giving CPL, the mouse was placed in a thin cylinder (diameter 2.7 cm, length 9.5 cm) for 19 hours (17:00 to the next morning 9:00) once a week, thereby confined space-restraint stress was given for 4 weeks (4 times). Immediately after completion of restraint, 0.005 ml of blood was collected from the caudal vein under diethyl etherized anesthesia, and the lymphocyte number was counted under the optical microscope.

### (Result)

The result of the counting of the lymphocyte number is shown in Table 4. As shown in Table 4, the lymphocyte count of the control group was reduced linearly until the 3rd week after applying the confined space-restraint stress was begun, to 30% of the value before the start of the experiment. At the 4th week, the value showed no change. In the CPL group, similar to the control group after the 1st confined space-restraint stress experiment, the reduction of the lymphocyte count was observed. Thereafter, the confined space-restraint stress experiments were repeated, but the lymphocyte count was maintained at an almost comparable count

**Table 4.**

| Comparison of the CPL group with the control group as to the change of lymphocyte count caused by the confined space-restraint stress | | | | | |
|---|---|---|---|---|---|
| | lymphocyte count ( × 10⁴/mm³) | | | | |
| Experiment period | Start | 1 week | 2 week | 3 week | 4 week |
| Control group | 33.6±10.7^{a)} | 26.5±4.3 | 17.7±7.1^{b)} | 10.8±2.0^{c)} | 12.1±4.9^{d)} |
| CPL group | 29.3±8.5 | 220±4.4 | 23.9±6.6 | 25.5±5.8 | 24.6±4.7 |

| | | | | | |
|---|---|---|---|---|---|
| a) mean ± Standard deviation Significant difference by t test in comparison with values at the start of the experiment for the control group: | | | | | |
| b) p<0.05 | | | | | |
| c) p<0.001 | | | | | |
| d) p<0.01 | | | | | |

### Test Eample 4: Oncogenesis suppressing effect of CPL under stress

The mice used in Test example 3 were the mice of a line occurring inherently alveolar epithelium hyperplasy. According to studies so far, tumor tissue gains energy depending on anaerobic glycolysis. It is recognized that CPL is a substance having the effect of suppressing the above oncogenesis by suppressing this glycolysis (56th Japan Cancer Association Annual Meeting Proceedings Article p. 605; Japan Cancer Therapy Association Journal 32(5): 165; 57th Japan Cancer Association Annual Meeting Proceedings Article p. 646; Japan Cancer Therapy Association Journal 33(3): 222). Therefore, mice were kept under the condition same as that of Test Example 3, and the effect of suppressing oncogenesis under the stress was studied.

As the experimental group, the control group (stress group) was allowed to take freely the standard solid diet CE2 (obtained from CLEA JAPAN INC.), and the stress CPL group was allowed to take freely the standard solid diet CE2 containing 0.1% by weight of CPL in the above described standard solid diet (prepared by CLEA JAPAN INC.). The confined space-restraint stress similar to that of Test Example 1 was applied to the aforementioned groups. In addition, the group (CE2 group) which received CE2 under the condition of no stress and the group (CPL group) which received CE2 containing CPL under the condition of no stress were used as the control groups.

The aforementioned 4 groups were kept up to the 30th week, euthanized at the 20th and the 30th weeks under diethyl etherized anesthesia to resect a lung. A tissue thin section was prepared to observe the proportion of alveolar epithelium hyperplasy by using the optical microscope. The result is shown in Tables 5 and 6. Table 5 shows the comparison of tumor regions of the lung at the 20th week after CPL administration was started. The result of observations of the tumor regions contained in a histopathological thin section of 6 animals per each group (5 to 8 sites an animal) is presented in percentage. Table 6 shows the comparison of tumor regions of the lung at the 30th week after CPL administration was started. The result of observations of the tumor regions contained in a histopathological thin section of 6 animals per each group (5 to 8 sites an animal) is presented in percentage.

**Table 5:**

| Comparison of tumor regions of the lung at the 20th week after CPL administration was started | | | | | |
|---|---|---|---|---|---|
| | Region (%) of alveolar epithelium hyperplasy in a histopathological thin section of lung | | | | |
| | 10% or lower | 10-30% | 30-50% | 50-80% | 80% or more |
| CE2 group | 36.1 | 25.0 | 33.3 | 2.8 | 2.8 |
| CPL group | 51.4 | 29.7 | 16.2 | 2.7 | 0.0 |
| Stress group | 21.1 | 26.3 | 13.2 | 23.7 | 13.2 |
| Stress CPL group | 44.4 | 30.6 | 16.7 | 8.3 | 0.0 |

**Table 6:**

| Comparison of tumor regions of the lung at the 30th week after CPL administration was started | | | | | |
|---|---|---|---|---|---|
| | Region of alveolar epithelium hyperplasy in a histopathological thin section of lung (%) | | | | |
| | 10% or lower | 10-30% | 30-50% | 50-80% | 80% or more |
| CE2 group | 42.4 | 12.1 | 27.3 | 12.1 | 6.1 |
| CPL group | 69.2 | 15.3 | 11.5 | 3.8 | 0.0 |
| Stress group | 25.7 | 2.9 | 11.4 | 34.3 | 25.7 |
| Stress CPL group | 41.2 | 17.6 | 14.7 | 11.8 | 11.8 |

From the result in Table 5, it is understood that in the mouse which was kept with CE2, the tumor region was enlarged under the stress condition. However, the mouse which was kept with CPL showed no difference between tumor regions in the presence and absence of the stress. The proportion of animals showing a more than 50% tumor region was increased from 5.4% to 36.9% by giving stress in the case where CE2 was taken, but this proportion was increased to only 8.3% by giving stress in the case where CPL was taken.

From the result in Table 6, it is understood that in the mouse which was kept with CE2 for 30 weeks, the proportion of animals showing more than 50% tumor region was 18.2% which was 3.25 folds the value of the 20th week. When the mouse received this diet and was kept under the stress condition, the proportion was increased to 60%, but this proportion was 23.6% when CPL was administered.

In addition, a sample prepared by homogenizing the lung of the mouse which was euthanized at the 30th week, was centrifuged to obtain a supernatant. The supernatant was used for measurement of enzyme activities of the anaerobic glycolysis, i. e., activities of hexokinase (HK), phosphofructokinase (PFK), pyruvate kinase (PK) and lactic acid dehydrogenase (LDH). Table 7 shows the result.

**Table 7.**

| Enzyme activities in the anaerobic glycolysis system at the 30th week | | | | |
|---|---|---|---|---|
| | HK | PFK | PK | LDH |
| CE2 group (no stress) | 75.5±7.7^{a)} | 48.3±4.5 | 224.0±28.5 | 1330±105 |
| Stress group | 91.7±4.9^{b)} | 64.0±4.4^{c)} | 274.0 ±32.0^{d)} | 1531±99^{b)} |
| Stress CPL group | 69.4±8.7 | 51.6±17.6 | 229.0±28.1 | 1364±172 |

| | | | | |
|---|---|---|---|---|
| a) mean ± Standard deviation Significant difference by t test in comparison of the CE2 group with the stress group: | | | | |
| b) p<0.01 | | | | |
| c) p<0.001 | | | | |
| d) p<0.05 | | | | |

As is understood from this result, the rate determining enzyme activity of the glycolysis metabolism in the lung was increased significantly by the confined space-restraint stress. However, each of these enzyme activities after CPL administration showed no significant difference to the CE2 group.

The results in Test Example 3 and Test example 4 have demonstrated that, by administration of CPL, the reduction of the lymphocyte count and the increase of the anaerobic enzyme activity due to the stress are suppressed, and anti-tumor effect is achieved.

### Industrial Applicability

The antistress agent according to invention can be used for relief and prevention of various stresses. Further, the mixture of poly lactic acids used in the present invention as an active ingredient is a low condensate of lactic acids derived from organism components, and therefore shows a high biocomparability and little side effects. Therefore, the antistress agent according to the present invention is excellent in safety, requires no special restriction for use, and can be used daily, for example as a beverage, in order to prevent and/or relieve the stress.

## Claims

1. An antistress agent which comprises a mixture of cyclic and/or straight chain poly lactic acids having a condensation degree of 3 to 20.

2. The antistress agent of claim 1 for preventing and improving a medical symptom caused by the stress.

3. The antistress agent of claim 1 or 2 for preventing and improving deterioration of an immune function which is caused by the stress

4. The antistress agent of claim 1 or 2 for preventing and improving an ulcer or a tumor caused by the stress.

5. The antistress agent of claim 1 or 2 for improving the survival ratio against the stress of a patient having a disease.

6. The antistress agent of any one of claims 1 to 5, wherein the lactic acid that is a repeating unit in the poly lactic acid consists substantially of L-lactic acid.

7. The antistress agent of any one of claims 1 to 6, wherein the mixture of cyclic and/or straight chain poly lactic acids having a condensation degree of 3 to 20 is a fraction obtained by condensing lactic acids by dehydration under an inactive atmosphere, subjecting the ethanol- and methanol-soluble fractions of the obtained reaction solution to reverse phase column chromatography, and eluting with 25 to 50 weight % acetonitrile aqueous solution of pH 2 to 3 and then with 90 weight % or more acetonitrile aqueous solution of pH 2 to 3.

8. The antistress agent of claim 7, wherein condensation by dehydration is performed by stepwise decompression and temperature rise under nitrogen gas atmosphere.

9. The agent of claim 7 or 8, wherein reverse phase column chromatography is performed by ODS column chromatography.

10. Food and drink for suppressing stress, which comprises the antistress agent of any one of claims 1 to 9.
